(19) Europäisches Patentamt
European Patent Office
Office européen des brevets

(11) EP 2 377 859 B1

(12) **EUROPÄISCHE PATENTSCHRIFT**

(45) Veröffentlichungstag und Bekanntmachung des Hinweises auf die Patenterteilung:
**04.03.2015 Patentblatt 2015/10**

(51) Int Cl.:
***C07D 401/04*** (2006.01)

(21) Anmeldenummer: **10157567.8**

(22) Anmeldetag: **24.03.2010**

(54) **Verbesserte Bis(triazin)-pyridin-Derivate und Verfahren zu deren Herstellung**

Improved bis(triazine)-pyridin derivatives and method for its production

Dérivées de bis(triazine)-pyridine améliorés et leur procédé de fabrication

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**19.10.2011 Patentblatt 2011/42**

(73) Patentinhaber: **Karlsruher Institut für Technologie
76131 Karlsruhe (DE)**

(72) Erfinder:
- **TRUMM, Sascha
76646, Bruchsal (DE)**
- **GEIST, Andreas
76344, Eggenstein-Leopoldshafen (DE)**
- **PANAK, Petra J.
76149, Karlsruhe (DE)**
- **FANGHÄNEL, Thomas
76356, Weingarten/Baden (DE)**

(74) Vertreter: **Fitzner, Uwe
Dres. Fitzner
Patent- und Rechtsanwälte
Hauser Ring 10
40878 Ratingen (DE)**

(56) Entgegenhaltungen:
- **HUDSON, MICHAEL J. ET AL: "New bis(triazinyl) pyridines for selective extraction of americium (III)" NEW JOURNAL OF CHEMISTRY , 30(8), 1171-1183 CODEN: NJCHE5; ISSN: 1144-0546, 2006, XP002586586**
- **GEIST, ANDREAS ET AL: "6,6'-Bis(5,5,8,8-tetramethyl-5,6,7,8-tetr ahydro-benzo[1,2,4] triazin- 3-yl)[2,2']bipyridine, an effective extracting agent for the separation of americium (III) and curium(III) from the lanthanides" SOLVENT EXTRACTION AND ION EXCHANGE , 24(4), 463-483 CODEN: SEIEDB; ISSN: 0736-6299, 2006, XP009134625**

Bemerkungen:
Die Akte enthält technische Angaben, die nach dem Eingang der Anmeldung eingereicht wurden und die nicht in dieser Patentschrift enthalten sind.

EP 2 377 859 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft neue alkylierte Bistriazinylpyridine und -bipyridine, ein Verfahren zu deren Herstellung sowie deren Verwendung.

1. Nach dem Stand der Technik sind verschiedene Extraktionsmittel bekannt, welche insbesondere dreiwertig vorliegende Aktiniden, vor allem Americium(III) und Curium(III) von den chemisch ähnlichen Lanthaniden aus Salpetersäurelösung durch Flüssig-Flüssig-Extraktion abzutrennen vermögen. So sind beispielsweise 2,6-Bis-(5,6-dialkyl-1,2,4-triazin-3-yl)pyridine der Formel I (BTP):

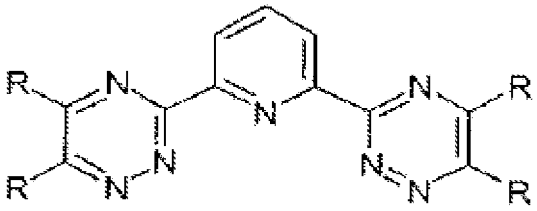

Formel I

aus der DE 19 180 895, Kolarik et al., Solvent Extr. Ion. Exch. 1999, 17 (1), 23-32 und 17 (5), 1155-1170 bekannt, welche für derartige Extraktionsverfahren geeignet sind. Allerdings hat die Praxis gezeigt, dass diese Mittel gegen Salpetersäurelösungen nicht ausreichend stabil sind.

Aus M.J. Hudson et al., New J. Chem. 2006, 30 (8), 1171 - 1183 sind modifizierte Moleküle mit einer verbesserten Stabilität gegenüber Salpetersäure bekannt. Es handelt sich hierbei um 2,6-Bis-(5,5,8,8-tetramethyl-5,6,7,8-tetrahydrobenzo[1,2,4]triazin-3-yl)pyridine der Formel II ($CyMe_4$-BTP):

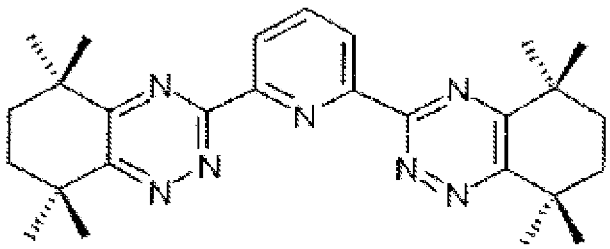

Formel II

Diese Mittel sind allerdings für die Praxis nicht einsetzbar, da eine Rückextraktion nicht möglich ist. D.h. eine Rezyklisierung des Extraktionsmittels ist nicht gewährleistet. Außerdem ist die Extraktionskinetik extrem langsam. Aus diesen Gründen ist ein technischer Einsatz dieser Mittel nicht möglich.

Aus M. R. St. J. Foreman et al., Solvent Extr. Ion. Exch. 2005, 23 (5) 645 - 662 sind ferner alkylierte 6,6'-Bis-(5,6-dialkyl-[1,2,4]triazin-3-yl)-2,2'-bipyridine der Formel III (BTBP):

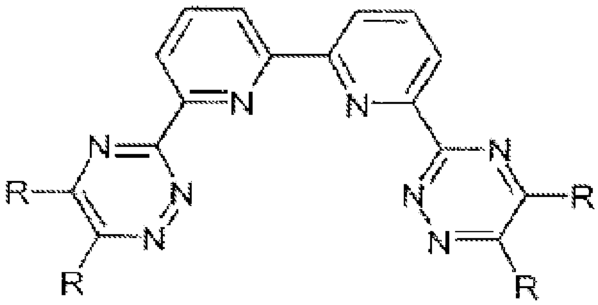

Formel III

bekannt. Aber auch diese alkylierten Bis-Triazinyl-Pyridine haben sich als nicht ausreichend stabil in Salpetersäure-Lösungen erwiesen.

Aus A. Geist et al. Solvent Extr. Ion. Exch. 2006, 24 (4) 463 - 483 ist ein 6,6'-Bis-(5,5,8,8-tetramethyl-5,6,7,8-tetrahydrobenzo[1,2,4]triazin-3-yl)-2,2'-bipyridin der Formel IV ($CyMe_4$-BTBP):

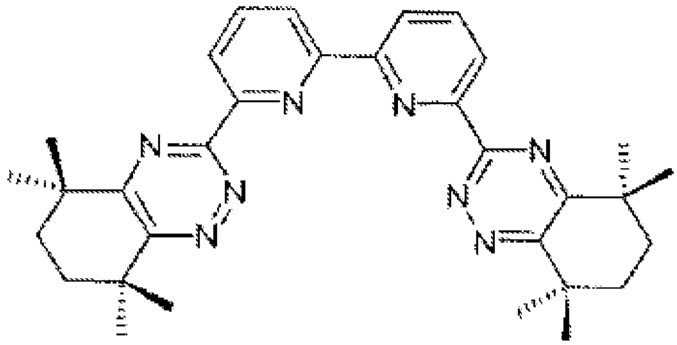

Formel IV

bekannt. Diese Verbindung zeichnet sich durch eine gute Extrahier- und Rückextrahierbarkeit aus. Allerdings ist die Reaktionskinetik für technische Zwecke nicht ausreichend schnell. Außerdem ist die Löslichkeit der Verbindung in den üblichen Verdünnungsmitteln zu niedrig.

Aus G. Modolo et al. Solvent Extr. Ion. Exch. 1999, 17 (1) 33 - 53 sind ferner aromatische Dithiophosphinsäuren der Formel V [$(ClPh)_2PSSH$)]:

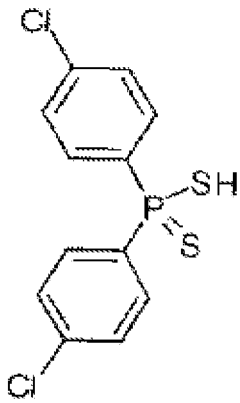

Formel V

in synergistischer Mischung mit neutralen Alkylphospaten oder Alkylphosphinoxiden der Formel VI (TOPO)

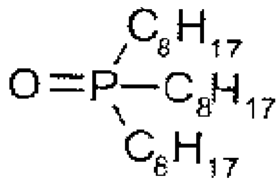

Formel VI

bekannt. Diese Verbindungen lassen sich in salpetersauren Lösungen einsetzen und weisen eine ausreichende Stabilität auf. Auch die Extraktionskinetik ist ausreichend schnell. Allerdings enthalten diese Verbindungen Heteroatome, so dass beim Verbrennen der Moleküle bzw. ihrer Abbauprodukte feste Abfälle entstehen. Dies ist ein Nachteil gegenüber Molekülen, die dem CHON-Prinzip folgen, bei denen ausschließlich gasförmige Produkte bei einer Verbrennung entstehen.

Die vorliegende Erfindung hat sich nunmehr die Aufgabe gestellt, ein Extraktionsmittel zur Verfügung zu stellen, welches dreiwertige Aktinide selektiv gegenüber den Lanthaniden aus salpetersaurer Lösung zu extrahieren vermag.

Diese Aufgabe wird durch ein modifiziertes Bistriazinylpyridin, zusätzliches Pyridin der Formel VII

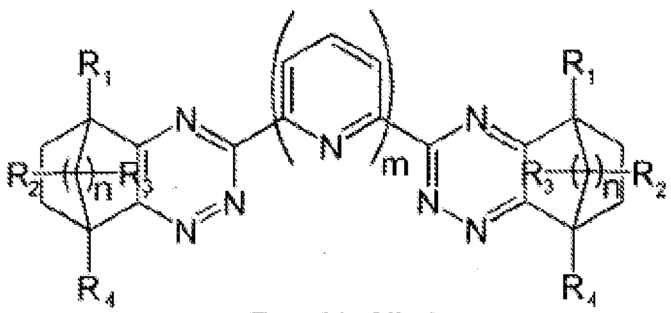

$R_n$ = H, Alkyl
m,n = 1,2

Formel VII

gelöst. Hierbei bedeuten: $R_{1/2/3/4}$= H, Alkyl; m = 1,2; n = 1,2

In einer bevorzugten Ausführungsform ist $R_n$=H, Methyl, Ethyl.

In einer weiteren bevorzugten Ausführungsform ist n=1,2. Weiterhin ist bevorzugt m=1,2.

In einer besonders bevorzugten Ausführungsform wird erfindungsgemäß ein Bistriazinylpyridin (2,6-Bis-(5,6,7,8-tetrahydro-5,9,9-trimethyl-5,8-methano-1,2,4-benzotriazin-3-yl)pyridin der Formel VIII (CA-BTB)

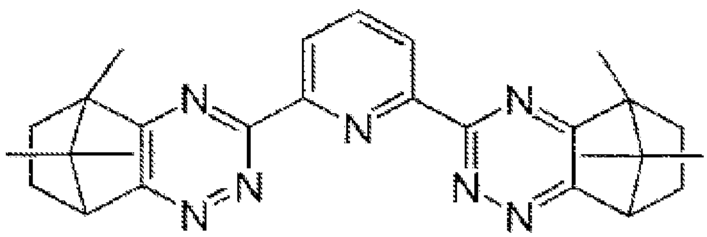

Formel VIII

eingesetzt.

Die erfindungsgemäß neu synthetisierten Verbindungen zeigen gegenüber den bisher bekannten Molekülen eine verbesserte Löslichkeit und deutlich schnellere Extraktionskinetik. Die Stabilität gegenüber Salpetersäure ist für technische Zwecke ausreichend. Außerdem sind Selektivität und direkte Extrahierbarkeit von Metallnitraten weitere vorteilhafte Eigenschaften.

Gegenstand der Erfindung ist ferner ein Verfahren zur Herstellung der erfindungsgemäßen Moleküle. Bei diesem Verfahren werden Pyridin-2,6-bisamidrazin mit Alkohol, vorzugsweise Ethanol und mit Campherchinon (1,7,7-trimethylbicyclo[2.2.1]heptan-2,3--dion) versetzt.

Bei den Racematen oder enantiomerenreinen Verbindungen der Formel IX

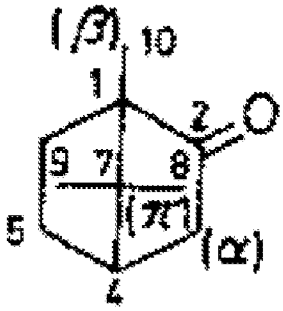

≡

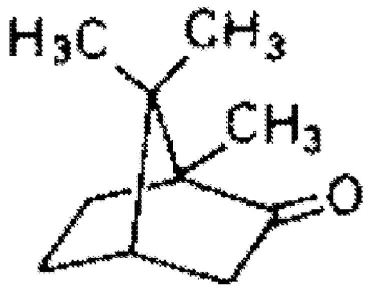

Formel IX

können Substituenten an den C-Atomen 4,8,9 und 10 eingesetzt werden. Auch diese Modifikationen sind erfindungsgemäß einsetzbar.

Das Gemisch aus Pyridin-2,6-bisamindrazin, Alkohol oder Tetrahydrofuran (THF), Campherchinon wird über mehrere Stunden, vorzugsweise 2,5 Stunden besonders bevorzugt 3 Stunden erhitzt. Im Anschluss daran wird weiter bei Raumtemperatur gerührt. Dies wird für 5 - 14 Stunden, bevorzugt 12 Stunden. Anschließend wird das Lösungsmittel entfernt. Hierfür wird vorzugsweise bei reduziertem Druck gearbeitet. Dieser beträgt 40 - 300 mbar , bevorzugt 200 mbar . Besonders bevorzugt ist die Entfernung im Vakuum. Nach Reinigung des Rückstandes, vorzugsweise mit chromatographischem Verfahren, wird ein Feststoff erhalten, bei welchem es sich um die Verbindung gemäß der Formel VII und VIII handelt.

Im allgemeinen werden 2 - 2,5 Teile Champherchinon und 1 Teil Pyridin-2,6-Bisamidrazin vermischt. Die für das Verfahren eingesetzten Mengen an Pyridin-2,6-Bisamidrazin betragen vorzugsweise 450 mg - 50 g, besonders bevorzugt 5 g. Der Campherchinon wird vorzugsweise in Mengen von 1 g - 100 g, besonders bevorzugt von 11 g verwendet.

Die Alkohole oder Ether werden vorzugsweise in Mengen von 10 ml - 1 l, besonders bevorzugt 100 ml eingesetzt.

Als Alkohole kommt vorzugsweise Ethanol zum Einsatz. Ebenso sind aber auch Methanol, Propanol und Tetrahydrofuran verwendbar.

Die beschriebenen Verbindungen eignen sich insbesondere als Extraktionsmittel im Rahmen der Reduktion der Langzeitradioaktoxizität abgebrannter Kernbrennstoffe. Insbesondere lassen sich dreiwertige Actiniden, vorzugsweise Americium(III) und Curium(III) selektiv gegenüber Lanthaniden aus salpetersaurer Lösung extrahieren. Demgemäß werden die Verbindungen vorzugweise für diese Zwecke verwendet.

Im Folgenden wird die Erfindung unter Bezugnahme auf die von den Erfindern durchgeführten Experimente näher dargelegt.

<u>Synthese</u>

Eine Suspension von Pyridin-2,6-Bisamidrazin (0,45 g, 2,33 mmol) in absolutem Ethanol (10ml) wurde mit (±)-Campherchinon (1 g, 5,13 mmol) versetzt und drei Stunden unter Rückfluss erhitzt. Anschließend wurde über Nacht bei Raumtemperatur gerührt und sodann das Lösungsmittel unter reduziertem Druck entfernt. Der Rückstand wurde blitzchromatographisch (Kieselgel, Petrolether:Essigester 1:1) gereinigt. Es wurden 591 mg (56 % d. Th.) CA-BTP als gelber Feststoff erhalten.

<u>Charakterisierung</u>

$^1$H NMR:

$\delta$ ($^1H$, $CDCl_3$) = 0,67 (s, 6 H, $CH_3$), 1.12 (s, 6 H, $CH_3$), 1.49 (s, 6 H, $CH_3$), 2.02-2.16 (m, 6 H, $CH_2$), 2.28-2.44 (m, 2 H, $CH_2$), 3.29 (d, 2 H, CH), 8.08 (t, 1 H, 4-H), 8.74 (d, 2 H, 3-H, 5-H)

ESI-MS:

m/z = 454 $[M+H]^+$, 476 $[M+Na]^+$, 907 $[3M+H]^+$, 929 $[3M+Na]^+$

Extraktionsversuche

Um die Eignung obiger Verbindung zur selektiven Extraktion dreiwertiger Actiniden zu untersuchen, wurde versucht, solvatisierte Metallnitrate gemäß

$$M^{3+}_{aq} + 3\ NO_3^-{}_{aq} + n\ \text{CA-BTP}_{org} = M(NO_3)_3\text{CA-BTP}_{n\ org}$$

zu extrahieren.

Experimentelles

Es wurden je 0,5 ml wässrige Phase ($^{24}$Am(III) + $^{152}$Eu(III) (je ca. 1 kBq/ml) in $HNO_3$) und 0,5 ml organische Phase (CA-BTP in Kerosin) 20 min. (bzw. unterschiedliche Zeitintervalle zur Bestimmung der Extraktionskinetik) auf einem Kresisschüttler bei 550 U/min kontaktiert. Nach Zentrifugation wurden je 0,3 ml wässrige bzw. organische Phase entnommen. In diesen Proben wurden auf einem Gamma-Counter $^{241}$Am und $^{152}$Eu bestimmt. Daraus ergibt sich das Verteilungsverhältnis als das Verhältnis der Zählraten in der organischen und in der wässrigen Probe,

$$D = cpm_{org}/cpm_{aq}$$

Der Trennfaktor ist das Verhältnis der Verteilungsverhältnisse von Americium und Europium also

$$SF_{Am(III)/Eu(III)} = D_{Am(III)}/D_{Eu(III)}$$

Zur Bestimmung der Stabilität des Liganden der organischen Phase wurde die organische Phase im Kontakt mit der wässrigen stehen gelassen und vor den Messungen für 15 min geschüttelt. Nach Zentrifugation wurden die Verteilungsverhältnisse wie oben beschrieben bestimmt. Anschließend wurden die Phasen wieder in Kontakt aufbewahrt.

Ergebnisse

Wie in Fig.1 gezeigt, erfolgt die Gleichgewichtseinstellung unter den gewählten experimentellen Bedingungen für CA-BTP bereits nach ca. 10 Min., während für $CyMe_4$-BTBP, das derzeitige Referenzmolekül, über zwei Stunden erforderlich sind. Dargestellt ist die Kinetik der Gleichgewichtseinstellung bei der Extraktion von Am(III) und Eu(III); Vergleich von CA-BTP (gefüllte Symbole) und $CyMe_4$-BTBP (offene Symbole). Wässrige Phase: $^{24}$Am(III) + $^{152}$Eu(III) je ca. 1kBq/ml in 1 M $HNO_3$. Organische Phase: 10 mM CA-BTP in Kerosin/1-Octanol 7:3 bzw. 10mM $CyMe_4$-BTBP in 1-Octanol. T= 20°C.

Die Abhängigkeit der Americium(III)- und Europium(III)-Verteilungsverhältnisse von der Salpetersäurekonzentration sind in Fig. 2 gezeigt: Americium(III) kann bei höheren Säurestärken mit guter Selektivität gegenüber Europium(III) - der Trennfaktor beträgt ca. 100 - extrahiert werden. Die Rückextraktion erfolgt bei niedriger Säurestärke.

Dargestellt ist die Abhängigkeit der Am(III) und Eu(III)-Verteilungsverhältnisse und des Trennfaktors von der Salpetersäurekonzentration. Wässrige Phase: $^{24}$Am(III) +$^{152}$Eu(III) (je ca. 1 kBq/ml) in 0,1-1 M $HNO_3$. Organische Phase: 50 mM CA-BTP in Kerosin.

Die Abhängigkeit der Americium(III)- und Europium(III)-Verteilungsverhältnisse von der CA-BTP-Konzentration sind in Fig. 3 gezeigt. Sie entspricht der Abhängigkeit, welche auch für andere BTP gefunden wurde und bestätigt die Extraktion von Americium(III) und Europium(III) gemäß Gl. S. 7, Z. 16, n=3. Die getestete maximale CA-BTP-Konzentration von 40 mM entspricht noch nicht der maximalen Löslichkeit in Kerosin.

Der Trennfaktor von ca. 100 ist im Wesentlichen von Salpetersäure- bzw. CA-BTP-Konzentration unabhängig.

Dargestellt ist die Abhängigkeit der Am(III)- und Eu(III)-Verteilungsverhältnisse und des Trennfaktors von der CA-BTP-Konzentration Wässrige Phase: $^{24}$Am(III) + $^{152}$Eu(III) (je ca. 1 kBq/ml) in 1 M $HNO_3$. Organische Phase: CA-BTP in Kerosin.

Die Stabilität gegenüber 1 M Salpetersäure ist in Fig. 4 gezeigt: Über einen Zeitraum von 113 Tagen bleiben die Extraktionseigenschaften (Verteilungsverhältnisse, Trennfaktor) praktisch konstant.

Dargestellt ist die Untersuchung der Stabilität gegenüber Salpetersäure. Wässrige Phase: $^{24}$Am(III) + $^{152}$Eu(III) (je ca. 1 kBq/ml) in 1 M $HNO_3$. Organische Phase: 30 mM CA-BTP in Kerosin.

Schlussfolgerung

Das neu synthetisierte CA-BTP zeigt im Vergleich zum derzeitigen Referenzmolekül $CyMe_4$-BTBP verbesserte Löslichkeit und deutlich schnellere Extraktionskinetik. Die Stabilität gegenüber Salpetersäure, die gute Selektivität sowie die direkte Extrahierbarkeit von Metallnitraten bleiben erhalten.

## Patentansprüche

1. Bistriazinylpyridin der Formel VII

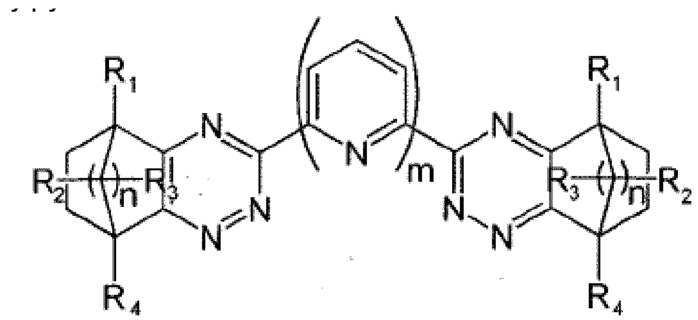

Formel VII

wobei $R_{1/2/3/4}$= H, Alkyl, m, n=1,2 bedeuten.

2. Bistriazinylpyridin gemäß Anspruch 1, **dadurch gekennzeichnet, dass** es 2,6-Bis-(5,6,7,8-tetrahydro-5,9,9-trimethyl-5,8-methano-1,2,4 benzotriazin-3-yl)pyridin der Formel VIII

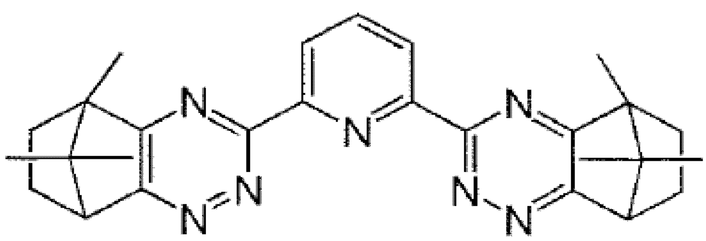

Formel VIII

ist.

3. Bistriazinylpyridin gemäß den Anspruch 1 **dadurch gekennzeichnet, dass** R = H, Methyl, Ethyl bedeutet.

4. Verfahren zur Herstellung der Bistriazinylpyridine nach einem der Ansprüche 1 bis 3 durch Mischen von Pyridin-2,6-bisamidrazin mit Alkoholen oder Ethern und Campherchinon.

5. Verfahren nach einem der vorhergehenden Ansprüche **dadurch gekennzeichnet, dass** Ethanol eingesetzt wird.

6. Verfahren nach einem der vorhergehenden Ansprüche **dadurch gekennzeichnet, dass** 450 mg - 50 g Pyridin-2,6-bisamidrazin und 1 g - 100 g Campherchinon eingesetzt werden.

7. Verfahren nach Anspruch 6 **dadurch gekennzeichnet, dass** 5 g Pyridin-2,6-bisamidrazin und 11 g Campherchinon eingesetzt werden.

8. Verfahren nach einem der vorhergehenden Ansprüche **dadurch gekennzeichnet, dass** das Gemisch für 2 - 5 Stunden auf 60 - 80 °C erhitzt, anschließend bei Raumtemperatur (15 - 25 °C) für 5 - 14 Stunden gerührt und anschließend das Lösungsmittel unter Druckreduzierung entfernt wird.

9. Verfahren nach Anspruch 8 **dadurch gekennzeichnet, dass** das Lösemittel bei einem Druck von 40 - 300 mbar entfernt wird.

10. Verwendung der Verbindung nach einem der Ansprüche 1 bis 3 als Extraktionsmittel.

11. Verwendung nach Anspruch 11 zur Abtrennung von Aktiniden.

## Claims

1. Bistriazinylpyridine of the formula VII

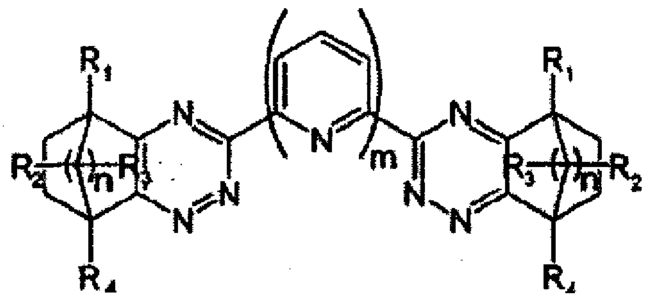

Formula VII

where $R_{1/2/3/4}$ = H, alkyl, m, n = 1, 2.

2. Bistriazinylpyridine according to Claim 1, **characterized in that** it is 2,6-bis(5,6,7,8-tetrahydro-5,9,9-trimethyl-5,8-methano-1,2,4-benzotriazin-3-yl)-pyridine of the formula VIII

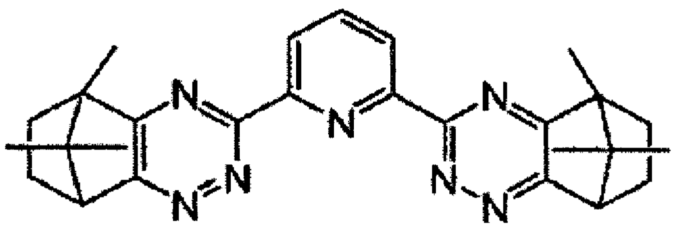

Formula VIII .

3. Bistriazinylpyridine according to Claim 1, **characterized in that** R = H, methyl, ethyl.

4. Process for preparing the bistriazinylpyridines according to any of Claims 1 to 3 by mixing pyridine-2,6-bisamidrazine with alcohols or ethers and camphorquinone.

5. Process according to any of the preceding claims, **characterized in that** ethanol is used.

6. Process according to any of the preceding claims, **characterized in that** 450 mg - 50 g of pyridine-2,6-bisamidrazine and 1 g - 100 g of camphorquinone are used.

7. Process according to Claim 6, **characterized in that** 5 g of pyridine-2,6-bisamidrazine and 11 g of camphorquinone are used.

8. Process according to any of the preceding claims, **characterized in that** the mixture is heated to 60-80°C for 2-5 hours, subsequently stirred at room temperature (15-25°C) for 5-14 hours and the solvent is subsequently removed under reduced pressure.

9. Process according to Claim 8, **characterized in that** the solvent is removed at a pressure of 40-300 mbar.

10. Use of the compound according to any of Claims 1 to 3 as extractant.

11. Use according to Claim 11 for separating off actinides.

## Revendications

1. Bistriazinylpyridine de formule VII

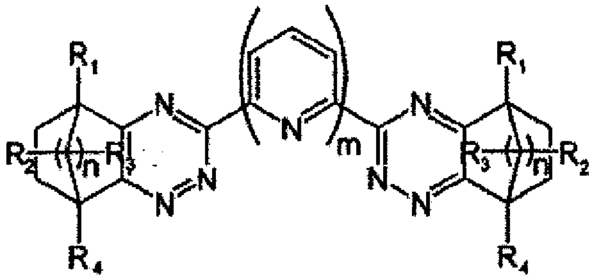

Formule VII

dans laquelle $R_{1/2/3/4}$ = H, alkyle ; m, n = 1, 2.

2. Bistriazinylpyridine selon la revendication 1, **caractérisée en ce qu'**il s'agit de la 2,6-bis-(5,6,7,8-tétrahydro-5,9,9-triméthyl-5,8-méthano-1,2,4-benzotriazin-3-yl)pyridine de formule VIII

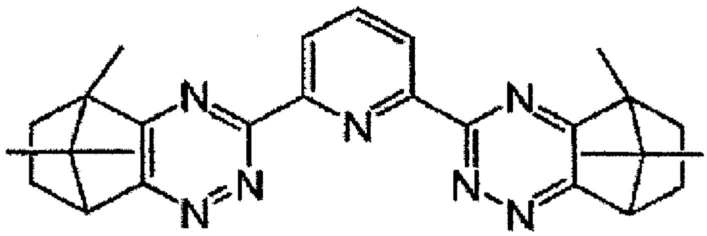

Formule VIII.

3. Bistriazinylpyridine selon la revendication 1, **caractérisée en ce que** R = H, méthyle, éthyle.

4. Procédé de fabrication des bistriazinylpyridines selon l'une quelconque des revendications 1 à 3 par mélange de pyridine-2,6-bisamidrazine avec des alcools ou des éthers et de la camphre-quinone.

**5.** Procédé selon l'une quelconque des revendications précédentes, **caractérisé en ce que** de l'éthanol est utilisé.

**6.** Procédé selon l'une quelconque des revendications précédentes, **caractérisé en ce que** 450 mg à 50 g de pyridine-2,6-bisamidrazine et 1 g à 100 g de camphre-quinone sont utilisés.

**7.** Procédé selon la revendication 6, **caractérisé en ce que** 5 g de pyridine-2,6-bisamidrazine et 11 g de camphre-quinone sont utilisés.

**8.** Procédé selon l'une quelconque des revendications précédentes, **caractérisé en ce que** le mélange est porté à une température de 60 à 80 °C pendant 2 à 5 heures, puis agité à température ambiante (15 à 25 °C) pendant 5 à 14 heures, puis le solvant est éliminé par réduction de la pression.

**9.** Procédé selon la revendication 8, **caractérisé en ce que** le solvant est éliminé à une pression de 40 à 300 mbar.

**10.** Utilisation du composé selon l'une quelconque des revendications 1 à 3 en tant qu'agent d'extraction.

**11.** Utilisation selon la revendication 11 pour la séparation d'actinides.

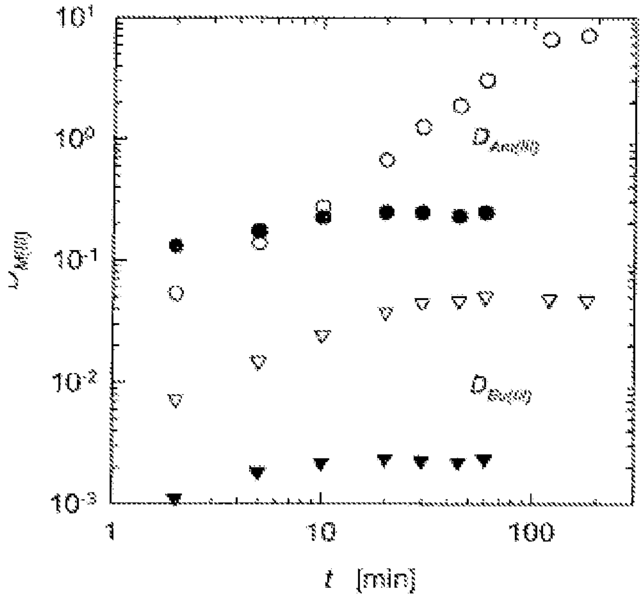
$D_{M(III)}$
$D_{Am(III)}$
$D_{Eu(III)}$
$t$ [min]

Fig. 1

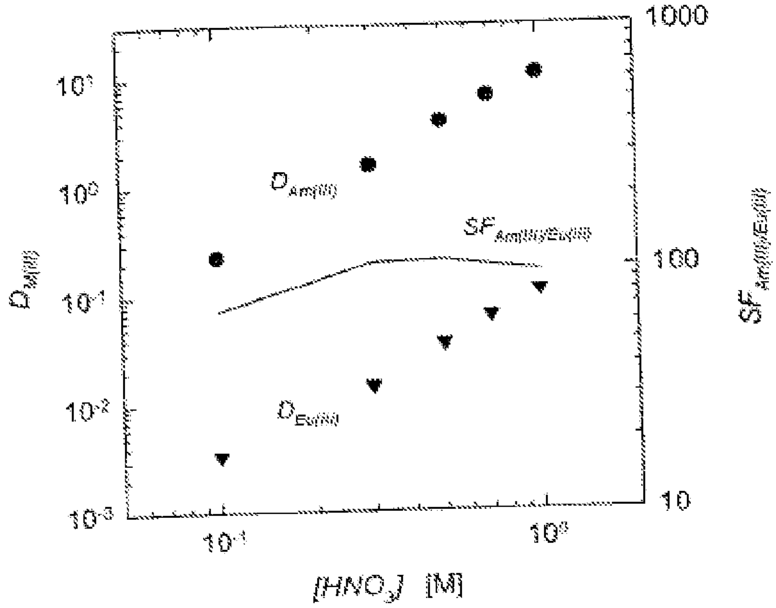
$D_{M(III)}$
$D_{Am(III)}$
$SF_{Am(III)/Eu(III)}$
$D_{Eu(III)}$
$[HNO_3]$ [M]

Fig. 2

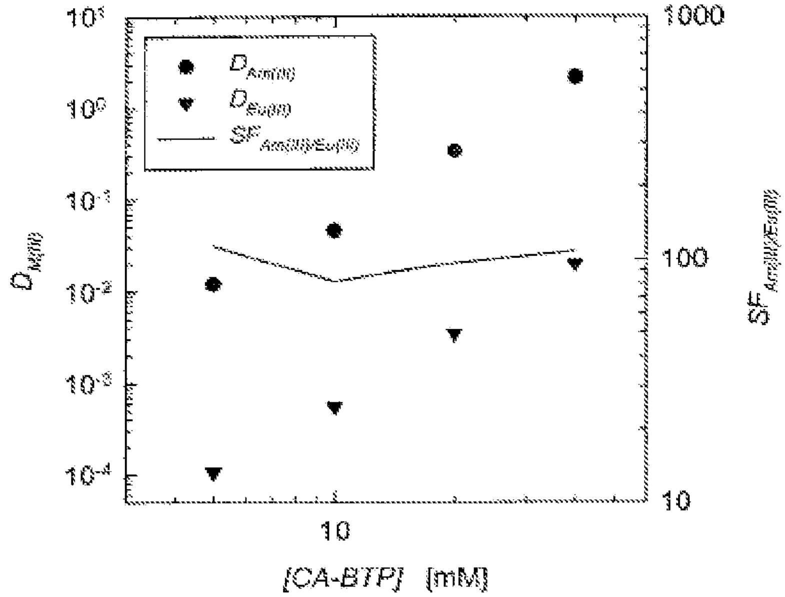


Fig. 3

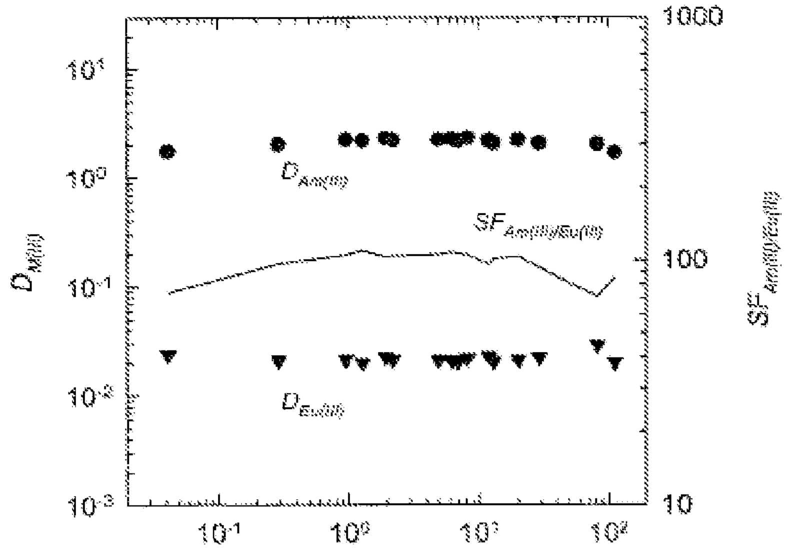


Fig. 4

Kontaktzeit [d]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19180895


### In der Beschreibung aufgeführte Nicht-Patentliteratur


- **KOLARIK et al.** *Solvent Extr. Ion. Exch.,* 1999, vol. 17 (1,5), 23-32, 1155-1170
- **M.J. HUDSON et al.** *New J. Chem,* 2006, vol. 30 (8), 1171-1183
- **M. R. ST. J. FOREMAN et al.** *Solvent Extr. Ion. Exch.,* 2005, vol. 23 (5), 645-662
- **A. GEIST et al.** *Solvent Extr. Ion. Exch.,* 2006, vol. 24 (4), 463-483
- **G. MODOLO et al.** *Solvent Extr. Ion. Exch.,* 1999, vol. 17 (1), 33-53